# EUROPEAN PATENT SPECIFICATION

(11) **EP 2 598 028 B1**
(45) Date of publication and mention of the grant of the patent: **18.01.2017**
(21) Application number: 11757939.1
(22) Date of filing: 12.07.2011
(51) Int. Cl.: A61B 5/11, A61N 1/37, G06F 19/00, A61N 1/365

(54) **AUTOMATIC ORIENTATION CALIBRATION FOR A BODY-MOUNTED DEVICE**
AUTOMATISCHE AUSRICHTUNGSKALIBRIERUNG FÜR EINE KÖRPERMONTIERTE VORRICHTUNG
ÉTALONNAGE D'ORIENTATION AUTOMATIQUE POUR UN DISPOSITIF MONTÉ SUR UN CORPS

(30) Priority: 27.07.2010 EP 10170857
(43) Date of publication of application: 05.06.2013
(73) Proprietor: Koninklijke Philips N.V., 5656 AE Eindhoven (NL)
(72) Inventor: VAN DEN HEUVEL, Teun, 5656 AE Eindhoven (NL); DURIC, Haris, 5656 AE Eindhoven (NL)
(74) Representative: van Velzen, Maaike Mathilde
(86) International application number: PCT/IB2011/053095
(87) International publication number: WO 2012/014110

(56) References cited:
- WO-A1-2009/112981
- WO-A2-2006/119344
- US-A1- 2007 115 277
- US-B1- 6 466 821
- US-B1- 7 149 584

## Description

### FIELD OF THE INVENTION

The present invention relates to a device, a system and a method for automatic calibration of an orientation of an implanted or body-mounted device relative to the body. Moreover, the present invention relates to posture detection of a user using the device.

### BACKGROUND OF THE INVENTION

As patient monitoring in general hospital wards and at home becomes more and more common, the circumstances, under which the monitored parameters are obtained, have also to be assessed automatically. For instance, the body posture has high influence on the heart rate, respiration rate and the like, so that these parameters should be evaluated dependent on the body posture of the patient. Furthermore, some medications or medical devices are applied depending on the activity or body posture of the patient. Thus, the body posture becomes an important parameter for medical monitoring or for the delivery of device assisted therapies, such as cardiac pacing, drug delivery and the like.

For determining a user's body posture, in general, three-dimensional accelerometers comprised in a body-mounted or implanted device are used. Accelerometers are not only sensitive to actual accelerations, but also to gravitational fields. As a result, in the absence of large accelerations, the output of an accelerometer reflects its orientation relative to the direction of the earth's gravitational field - at least for accelerometer types that are not only sensitive in a high frequency range. Hence, the DC component of the accelerometer output is highly dominated by gravity. Accelerations are popularly measured in terms of g-force. Thus, an accelerometer at rest relative to the earth's surface will indicate approximately 1g upwards, because any point on the earth's surface is accelerating upwards relative to the local inertial frame. Preferably, DC accelerometers are used for determining the orientation with respect to the earth's surface, and in particular DC accelerometers having a superior performance in the low frequency range for determining the orientation of the accelerometer, e. g. capacitive accelerometers, small micro electro-mechanical systems (MEMS), piezo-resistive accelerometers, etc..

Accelerometers attached to parts of the human body thus provide information related not only to accelerations of those parts, but also to their orientation with respect to gravity. These orientations can be used to describe posture, e.g. standing, sitting, lying supine, lying prone, etc. The most discriminative part of the human body in terms of body posture is the trunk or torso, i.e. the thorax and abdomen combined.

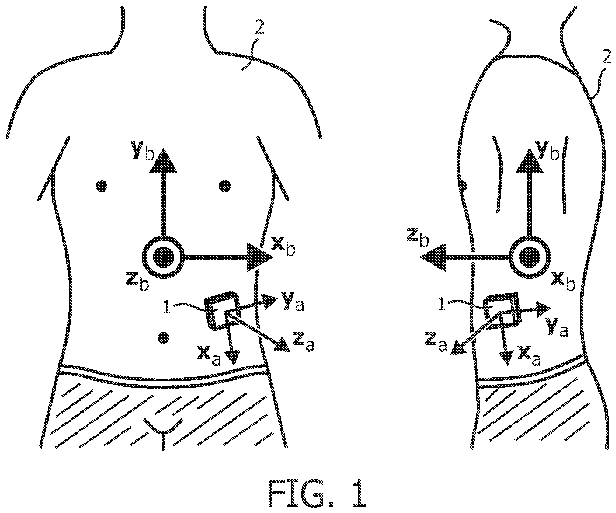

However, in order to determine a body posture, the orientation of the device incorporating a three-dimensional accelerometer relative to the body has to be determined first. As shown in Figure 1, three-dimensional coordinate systems defined for the trunk of the human body (b-system: x_{b}, y_{b}, z_{b}) and the accelerometer or accelerometric device attached to the body (a-system: xₐ, yₐ, zₐ) are in general not aligned and have different orientations. This may either be due to the body curvature, e.g. curvature of the chest, or to placement inaccuracy. For determining the orientation of the accelerometer coordinate system (a-system: xₐ, yₐ, zₐ) relative to the body coordinate system (b-system: x_{b}, y_{b}, z_{b}) with sufficient reliability for the degree of detail required by the respective application, four categories of calibration are generally known: 1. visual inspection, 2. additional measurements, 3. calibration procedures and 4. calibration from accelerometer output.

The first calibration methods take place during or after the attachment of the accelerometric device to the body, wherein the relative orientation of the device may be assessed by visual inspection. This requires a skilled person to be present and time to be reserved. Moreover, the levels of accuracy and precision of these methods may not be sufficient for most applications.

As an alternative to visual inspection, some additional measurement devices may be employed to quantitatively determine the relative device orientation, e.g. angle and distance measurements or camera observation. This category specifically requires the availability and skilled application of such additional devices, as well as available time (in most cases). Another possibility is to assume sufficient alignment. However, this requires skill and attention during placement. Also, a perfect placement is often not possible due to body curvatures and the like. Thus, deviations may result in consistent posture misidentification.

For the third kind of calibration procedures, the person subjected to measurement may be asked to subsequently adopt a set of various clearly defined postures, while the accelerometric device is attached to its body. With the known set of postures and the accelerometric output recorded during this procedure, the relative device orientation can be determined reliably enough for the discrimination of at least the postures in that procedure. The requirements of the methods in this category, however, include the subject to be cooperative and able to adopt a range of postures, and, again, time to be available.

A final category may be defined for methods that do not require any additional resources, i.e. only use the uncontrolled accelerometer output over a period of time to determine the relative device orientation. Different accelerometric signal features may be used to identify posture specific characteristics. Additionally, these methods may incorporate environment and subject specific knowledge, such as the typical prevalence of different postures. The present invention belongs to this category.

Another method belonging to this category is described in U.S. Patent no. 6,044,297. In this method, so called activity counts (accelerometric peaks exceeding certain thresholds) are detected. It is assumed that these counts only occur during an upright posture. The method furthermore assumes that detection of the upright posture constitutes sufficient device orientation calibration, because either the device attachment is such that no further calibration is needed, or a more detailed discrimination of non-upright postures is not required. Moreover, the accelerometric peaks primarily relate to periodic accelerations in moderate to intensive physical activities, e.g. walking, running and cycling. This method is therefore less suitable for the assessment of lighter types of physical activity, such as activities in a chair or a bed.

US 2007/0115277 A1 describes posture detection. A combination of two DC accelerometers and one AC accelerometer is used to determine a vector in a reference system of a medical device, wherein the gravitational acceleration G is used as a reference for determining said vector. Then, by using a transfer matrix determined during a calibration process, said vector is converted into body coordinates.

### SUMMARY OF THE INVENTION

One object of the present invention is to provide a device, a system and a method adapted to reduce requirements for personnel, time and/or additional equipment resources associated with existing devices and device orientation calibration methods in posture detection applications, while providing means for an accurate, inexpensive and fast detection of the device orientation relative to the body in three dimensions. It is a further object of the present invention to determine the body posture of the subject.

The objects are solved by the features of the independent claims. More precisely, the invention is based on the insight that postures in orientation data measured by an orientation detection unit are not only identifiable by their corresponding direction of gravity, but also by some other characteristics. Thus, an uncontrolled output of an orientation detection unit over a period of time together with one or more reference conditions defined in a body reference system should be sufficient for determining the relative orientation of the orientation detection unit and hence for calibration.

In one aspect, an implantable or body-mountable device is provided capable of determining the device orientation relative to the body. The device comprises a three-dimensional (i.e. three-axial) orientation detection unit that can measure orientation data for each of its three axes with respect to its reference system. In addition, the device comprises a control unit in order to determine the orientation of the device relative to the body by means of the measured orientation data and at least one predetermined reference condition defined in a body reference system. Thus, in a most preferred embodiment, the orientation detection unit measures orientation data in its reference system without any condition or assumption. The control unit may apply the at least one reference condition defined in the body reference system to the measured orientation data and identify or calculate a first and a second axis of the body reference system in the measured orientation data by means of the reference condition. Thus, the control unit can determine the orientation of the device relative to the body by determining a transformation relation between the body reference system and the reference system of the orientation detection unit by means of the reference condition and the measured orientation data.

Preferably, the device comprises further a communication unit for transmitting and/or receiving signals via wired or wireless communication. For instance, the device may transmit raw and/or processed orientation data and/or a determined relative device orientation to an external device, e. g. a computer, a PDA, a mobile communication terminal, etc. Moreover, the device may comprise a memory for storing data, a determined relative device orientation, reference conditions, constraints, assumptions etc..

The reference condition should allow the determination of a unique relation between the reference system of the orientation detection unit and the body reference system. Such a reference condition may relate to a certain activity pattern associated with a body posture, a certain prevalence level for a body posture considering the situation or the physical condition of the user, the absence or negligible likelihood for certain body postures (upside down), etc. Preferably, the reference condition is set according to the measurement circumstances or application. By defining a reference condition in the body coordinate system, a relation is defined between the data measured in the reference system of the orientation detection unit and the body coordinate system. Of course, the order of the steps of defining a reference condition in a body reference system and of measuring orientation data in the reference system of the orientation detection unit may be interchanged. Thus, according to the present invention, only the output signals of the three-dimensional orientation detection unit comprised in a body-mounted or implanted device and general information about the measurement situation are used to automatically calibrate the three-dimensional orientation of the device relative to the body. The resulting advantage is that posture detection can be applied without strict requirements for personal, time and/or additional equipment.

In order to determine the orientation of the device relative to the body, a transformation relation between the body reference system and the reference system of the orientation detection unit may be determined. For this, it may be assumed that the origins of both coordinate systems coincide. In this case, a translation from one coordinate system to the other will refer to a rotation. Moreover, the determined translation relation may be stored in order to calibrate orientation data for determining a body posture.

The orientation detection unit may comprise a three-dimensional accelerometer or any other three-dimensional device capable of determining its position relative to the gravitational field. "Three-dimensional" refers here to having three sensitive axes, so that a value is determined for each of these axes. The three values are then combined to indicate a point in a three-dimensional coordinate system.

Preferably, the reference condition comprises a reference posture or a reference posture range in the body coordinate system, which has at least one predetermined characteristic. For instance, a reference posture may be standing upright, lying supine, etc. An example for a reference posture range may be all positions of the transition from lying supine to standing upright or sitting up. The predetermined characteristic of the reference posture or reference posture range may relate to a particularly high or low prevalence of this posture or posture range and/or to a specific activity pattern. For instance, it may be assumed that an upright posture is accompanied with higher activity than other postures. Also, with respect to the situation of the user, certain postures may be more likely and have a higher prevalence than other postures. For instance, a bedridden person will spend most of his time lying down. Hence, by defining the measurement circumstances in one or more reference conditions, the calibration or interpretation of the measured data is simplified.

In a preferred embodiment, the control unit is further adapted to identify the reference posture or reference posture range in the measured orientation data in the reference system of the orientation detection unit by means of the predetermined characteristic. For instance, if the reference condition refers to an upright posture with high activity, a cluster of data points with high activity may be identified as related to the upright posture. By these means, one of the axes of the body reference system can be aligned with the respective axis of the reference system of the orientation detection unit. For some applications, it may already be sufficient to identify the relative position of the body-mounted device along one axis.

Preferably, at least one further constraint or assumption can be defined in addition to the reference condition for accelerating and simplifying the orientation determination process. For example, it may be assumed that certain postures will be completely absent (standing upside down), that a certain reference posture or reference posture range is associated with a certain prevalence and/or activity, etc..

Furthermore, the control unit may be adapted to calculate statistical parameters of the measured orientation data, e.g. an average of the measured orientation data for a predetermined time interval. The average (or mean or median) of the data in this time interval may then be stored or plotted as point in the reference system of the orientation detection unit. This time interval may be adjusted according to the application. Furthermore, the time intervals may be overlapping and/or having different sizes. Preferably, also the variance and/or covariance and/or standard deviation of the measured orientation data in this time interval is determined in order to estimate the occurrence and intensity of posture changes and thus the activity of the user.

In another preferred embodiment, the control unit may be adapted to perform cluster analysis on the measured orientation data, since it can be assumed that typical postures will occur in clusters of data points. Cluster analysis may be performed based on hierarchical, density-based, correlation-based or other algorithms. For instance, a cluster may be defined by a maximal distance between data points in a reference system.

In one embodiment, the measured orientation data are processed separately for each axis of the orientation detection unit resulting in three-dimensional values, indicating a point in the three-dimensional reference system.

Preferably, rapidly changing postures are filtered in the data analysis by setting a maximal spatial distance between subsequent data points. Thus, posture transitions will not be misinterpreted.

Preferably, the control unit may be adapted to repeat the determination of the device orientation relative to the body in predetermined time intervals and/or when it is triggered by a signal or special event. This is particularly advantageous, since an implanted device may change its position relative to the body on the long scale. It is even more important for a body-mounted device that may be taken off and thus changes its position every time it is put on again. However, it is strongly preferred to attach the device to the body such that it is not likely to change its position during measurements, since it is assumed that the orientations of the body reference system and the reference system of the orientation detection unit are time-invariant. Events triggering a new calibration can be, for example, switching on the device, attaching the device to the body, operating an input unit and the like. Alternatively or additionally, the calibration is performed periodically and/or at fixed points in time, for instance twice a day or each morning. By these means, the accuracy of the determined device orientation relative to the body is ensured and remains very reliable.

In all embodiments, orientation data may be measured continuously or in certain intervals. This may be set before the device is used.

In a particularly preferred embodiment, the device is applicable for determining a body posture. For this, the determined relative device orientation is used for translating the measured orientation data measured in the reference system of the orientation detection unit into the body reference system for obtaining body postures with respect to the earth's gravitational field (i.e. absolute body postures). The determined body posture may be used in clinical alarm or information systems, for giving posture-related feedback to a user (e.g. when doing physical exercises) and/or for controlling a pacemaker and/or device assisted drug delivery or the like. For instance, when it is determined that the user is standing upright or walking, a pacemaker may be regulated to have a higher frequency.

Preferably, the body posture is determined in real-time and/or continuously. Alternatively, it may also be set that the body posture is determined with certain time intervals or at certain points in time. Moreover, the body posture may also be determined off-line, i.e. after recording the orientation data. However, for some applications, the body posture may be irrelevant and only the relative orientation of the body-mounted device with respect to the body may be important. An example for such an application is the monitoring of the position or orientation of an implanted device.

In a further aspect, a system is provided for determining an orientation of a device relative to a body, wherein the device is implantable or mountable to the body and comprises a three-dimensional orientation detection unit capable of measuring orientation data for each of its three axes in a reference system corresponding to the orientation detection unit. Moreover, the system comprises a control unit that can determine the relative device orientation with respect to the body using the measured orientation data and a predetermined reference condition defined in a body reference system. The control unit and the device are capable of wired and/or wireless communication for transmitting data and signals in one or both directions. Preferably, the device is configured according to any one of the above-described embodiments.

In another aspect, a method is provided for calibrating a posture detecting device by determining an orientation of the device relative to a body, the device being mounted to or implanted in the body and comprising a three-dimensional (i.e. three-axial) orientation detection unit, wherein at least one reference condition is defined in a body reference system, orientation data are measured for each of the three axis of the orientation detection unit in a reference system of the orientation detection unit and the orientation of the device relative to the body is determined by means of the reference condition and the measured orientation data. Alternatively, a method is provided for calibrating a posture detecting device by determining an orientation of the device relative to a body, the device being mounted to or implanted in the body and comprising a three-dimensional orientation detection unit, wherein the method comprises the following steps: obtaining orientation data measured by the orientation detection unit in a reference system of the orientation detection unit; applying at least one reference condition defined in a body reference system to the measured orientation data; identifying a first and a second axis of the body reference system in the measured orientation data by means of the reference condition; and determining the orientation of the device relative to the body by determining a transformation relation between the body reference system and the reference system of the orientation detection unit by means of the reference condition and the measured orientation data. Here, it should be definite that the two axes are identified or calculated and not just defined by an assumption. Moreover, the data are measured without any reference condition.

In a further aspect, a computer readable medium is provided that can be executed on a computer in order to process orientation data measured by an implantable or body-mountable device having a three-dimensional orientation detection unit for determining a relative orientation of the device with respect to the body according to any one of the preceding embodiments of the invention.

### BRIEF DESCRIPTION OF THE DRAWINGS

Further features and advantages of the present invention will arise from the detailed description of embodiments with reference to the enclosed drawings.
Fig. 1 illustrates a reference system (xₐ, yₐ, zₐ) of a body-mounted device with respect to a body reference system (x_{b}, y_{b}, z_{b}).
Fig. 2 shows projections of y_{b} in the xₐ-yₐ-plane and the yₐ-zₐ-plane.
Fig. 3 shows measured orientation data represented as data points in a three-dimensional coordinate system.
Fig. 4 shows the measured orientation data with the upright body posture being identified and aligned with the direction perpendicular to the plane of projection.
Fig. 5 shows the alignment of the measured orientation data for the horizontal postures.
Fig. 6 shows the measured orientation data transformed into the body reference system, wherein five different postures are distinguished.
Fig. 7 is a flow diagram illustrating a preferred embodiment of the method according to the invention.
Fig. 8 is a flow diagram illustrating an embodiment of the "create map" sub-process of Fig. 7.
Fig. 9 is a flow diagram illustrating an embodiment of the "general cluster detection" sub-process of Fig. 8.
Fig. 10 is a flow diagram illustrating an embodiment of the "high activity cluster detection" sub-process of Fig. 8.
Fig. 11 is a flow diagram illustrating an embodiment of the "identify y_{b} in a-system" sub-process of Figure 8.
Figure 12 is a flow diagram illustrating an embodiment of the "identify x_{b} in a-system" sub-process of Figure 8.
Figure 13 illustrates estimating the location of x_{b} as part of the "identify x_{b} in a-system" sub-process according to Figure 12.

### DETAILED DESCRIPTION OF EMBODIMENTS

In Figure 1, a device 1 is shown that is mounted or implanted to a body 2. For instance, the device may be integrated or comprised in an implant, e.g. a pacemaker, a drug delivery control device, a patient monitoring device for monitoring a respiratory rate, heart rate and/or blood pressure, and the like. Alternatively, the device may be fixed around the thorax or abdomen using straps or the like or be integrated in another device, e.g. a heart or respiration rate monitoring device. The device comprises a three-dimensional orientation detection unit, e. g. a three-dimensional accelerometer. "Three-dimensional" refers here to sensing along three axes of the orientation detection unit. The orientation detection unit is fixedly comprised in the device 1, so that a reference system of the orientation detection unit (a-system: xₐ, yₐ, zₐ) can be regarded as a reference system of the device 1. As shown in Figure 1, the a-system of the device 1 is not aligned with a body reference system (b-system: x_{b}, y_{b}, z_{b}). In the example shown, the b-system (x_{b}, y_{b}, z_{b}) is chosen such that y_{b} points from the torso upwards to the head, x_{b} points from the centre of the torso to the left arm and z_{b} represents the third axis orthogonal to the other two axes. However, the choice of the reference systems and the choice of the orientation of their axes are arbitrary. In order to determine the relative orientation of the device 1 with respect to the body 2 and for determining a body posture, orientation data measured in the a-system (xₐ, yₐ, zₐ) of the device 1 have to be transformed into the b-system (x_{b}, y_{b}, z_{b}) of the body 2. It is preferred that the device 1 is mounted such that the tilt up/down of the accelerometer is less than 45° with respect to axis y_{b} of the body reference system.

As mentioned before, the orientation detection unit may comprise or may be realized as a three-dimensional accelerometer, e. g. a piezo-resistive accelerometer, a capacitive accelerometer, or other accelerometers, which have a sufficiently high sensitivity for low frequencies. However, instead of an accelerometer, any other means can be used that is capable to determine its three-dimensional orientation with respect to the earth's gravitational field. Without loss of generality, the functions of an orientation detection unit according to the invention are described here for an accelerometer and in the following, it is referred to an orientation detection unit as an accelerometer. Moreover, "posture" is generally defined as the direction of gravity with respect to the body reference system (x_{b}, y_{b}, z_{b}). Postures are represented as points on the unit g-sphere, since at rest, an accelerometer will output 1g. Moreover, it is assumed that the a-system (xₐ, yₐ, zₐ) of the device 1 has a time-invariant relative orientation with respect to the b-system (x_{b}, y_{b}, z_{b}) of the body 2 - at least for sufficiently long time scales. The origins of both reference systems are considered to be identical in accelerometric terms. In other words, the b-system (x_{b}, y_{b}, z_{b}) refers to the reference system of the torso and the device 1 is implanted or attached to the torso such that the device 1 is substantially immobile with respect to the torso.

The implantable or body-mountable posture-detecting device 1 preferably comprises a control unit adapted to perform auto-calibration by determining the relative device orientation with respect to the body 2 as described below. Furthermore, the device 1 can comprise a communication unit for exchanging signals or data with an external device, e.g. a computer, PDA, monitoring device or control unit. For instance, the device can transmit or receive orientation data measured by the accelerometer, processed orientation data, a determined relative device orientation, parameters such as time intervals, conditions, assumptions, constraints and the like. A user or therapist may input settings, parameters, etc. by means of the external device. Thus, the device 1 can be integrated in clinical alarm or information system, in a patient monitoring system or in a pacemaker or drug delivery system, wherein either the determined relative orientation of the device 1 with respect to the body 2 or a determined body posture is used. Moreover, the implantable or body-mountable device 1 may comprise a memory for storing measured or processed orientation data, reference conditions, parameters and the like.

Alternatively to a device 1 comprising an accelerometer, a control unit, storage means and a communication unit, the posture-detecting device 1 may also only comprise an accelerometer and a communication unit for wired or wireless communication with an external control unit, such as a computer, PDA or microcontroller. Storage means may either be provided in the device 1 or in the external control unit. This embodiment has the advantage that the dimensions and the weight of the device 1 as well as its production costs can be reduced. However, the flexibility in the device applications is reduced, since posture detection or calibration can only be performed, when communicating with the external control unit. In the system, an alarm unit may be included that raises an alarm, e.g. if a monitored heart rate exceeds a certain threshold set for the body posture determined by the device 1.

According to the basic idea of the present invention, postures in accelerometric signals are not only identifiable by their corresponding direction of gravity, but also by some other characteristics. Two important characteristics are (1) their prevalence and (2) their associated physical activity levels. Both of these characteristics may differ with subject and environment conditions.

As stated before, an accelerometer's output generally only indicates the direction of gravity in the absence of large accelerations. However, when accelerated, the accelerations correspond to accelerations of the body part, to which the device 1 is attached, and thus, the accelerations correspond to aspects of the user's physical activity. As such, measures for the signal amplitude of the measured orientation data in higher frequency ranges are known to provide a clear indication of a subject's physical activity level. A common measure of signal amplitude in this context is signal variance.

In most situations, different postures can be expected to have a different prevalence. Depending on the situation, some postures may be rare (e.g. leaning forward) or even completely absent (e.g. standing upside-down). Such information, if available in advance, may be used in an automatic device orientation calibration method. In the more common postures, it may be expected that the physical activity level is typically higher or lower than average in specific (groups of) postures. Specifically, in most situations, the horizontal postures (i.e. lying supine, lying on a side, lying prone) would typically be associated with lower levels of physical activity, whereas the upright posture would be associated with higher levels of physical activity. The latter association may still be distinguishable even when the subject is not walking, running or cycling, but merely sitting up. This effect can be attributed to the fact that the human body is commonly less well supported and therefore less stable in the upright posture, compared to other postures. This reduced stability is reflected in a higher activity level, at least in terms of accelerometric signal variance.

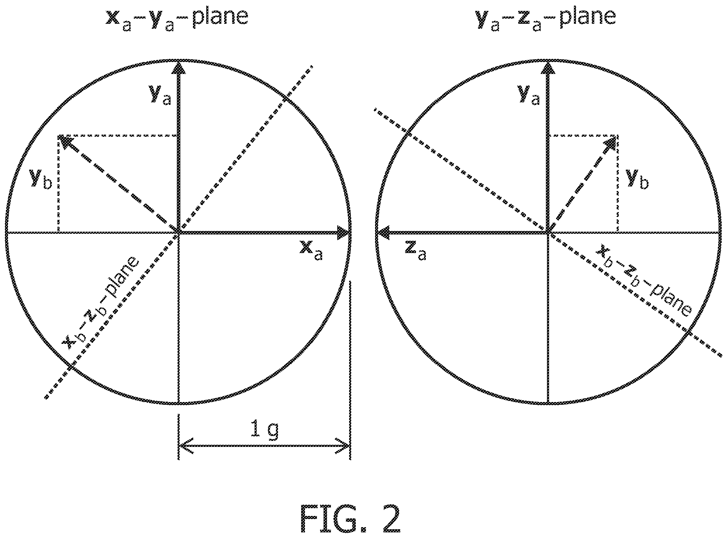

Figure 2 illustrates projections of y_{b} in the xₐ-yₐ-plane and the yₐ-zₐ-plane, respectively, represented as dashed arrows. Since at rest, the acceleration is 1g, a posture at rest will be on the surface of a unit sphere with radius 1g. Also shown are the lines of intersection of the x_{b}-z_{b}-plane with the xₐ-yₐ-plane and the yₐ-zₐ-plane, respectively. By definition, this x_{b}-z_{b}-plane is perpendicular to y_{b} and intersects the axis at the origin. A similar diagram could be drawn for the xₐ-zₐ-plane, but it would not contain any additional information.

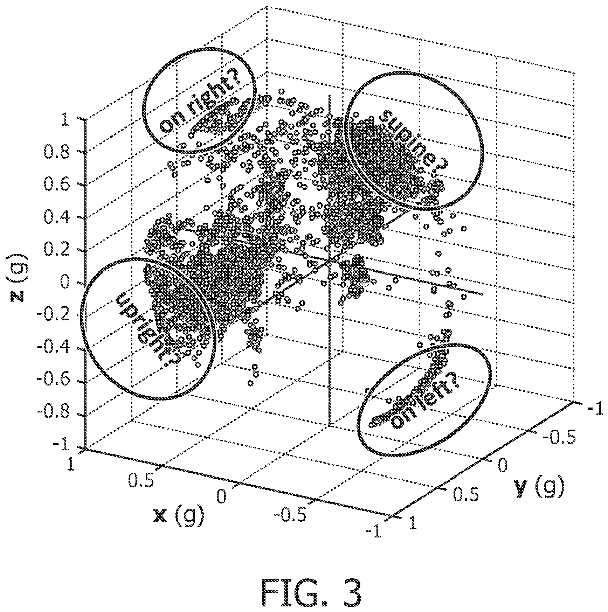

In Figure 3, full length orientation data measured by the accelerometer included in the body-mounted device 1 for about three hours are shown as signal average per second in a three dimensional coordinate system. It can be assumed that typical postures will occur as clusters. Moreover, the relative orientation of those clusters or postures is fixed. For instance, if the upright posture corresponds to (0, 1, 0) and the supine posture to (0, 0, 1) in the b-system, respectively, the coordinates (1, 0, 0) correspond to lying either on the left or on the right, depending on definition. In addition, further assumptions with respect to the prevalence of postures can be made: For example, upside down postures are not expected and certain postures as lying on the left or on the right will be less frequent than an upright or supine posture. In fact, this may already suffice to find the translation relation between the a-system (xₐ, yₐ, zₐ) of the device and the b-system (x_{b}, y_{b}, z_{b}) of the body, if the measurement time has been long enough to discern clusters and if a large range of different postures has been adopted. However, in order to speed up the calibration, i.e. the determination of the relative device orientation, the following embodiment of the calibration method according to the present invention is proposed.

First, a reference condition is defined in the b-system (x_{b}, y_{b}, z_{b}). In the present example, it is assumed that the upright posture will be of particularly high activity. Then, full-length orientation data measured by the accelerometer are recorded for a sufficiently long time. The measured data can be averaged for a predetermined time interval, e.g. per second, as shown in Figure 3. Furthermore, activity information may be obtained for each averaged data point by calculating the variance, covariance, standard deviation or the like. Preferably, the variance of the measured orientation data is calculated for a certain time interval. Considering now the reference condition, a unique cluster of high activity is identified as upright posture. The data may then be transformed such that the cluster of high activity is aligned with the axis of the b-system (x_{b}, y_{b}, z_{b}) corresponding to the upright posture, in the example shown y_{b}. For instance, this can be performed by taking the average, the median or the centre of the high activity cluster and rotating all orientation data points, until the centre of the high activity cluster is located on the y_{b}-axis of the body reference system.

Clusters may be defined by setting a minimal average (or median) distance between points, wherein points that are not part of the cluster may be left out in the further analysis. The centre of a cluster can be found by taking the average or median of points corresponding to this cluster. Points with high activity level can be identified by setting a minimal activity level, i.e. a minimal standard deviation or variance. Possibly, rapidly changing postures are left out in the analysis in order to accelerate and simplify the procedure. This can be achieved by setting a maximal spatial distance between subsequent points.

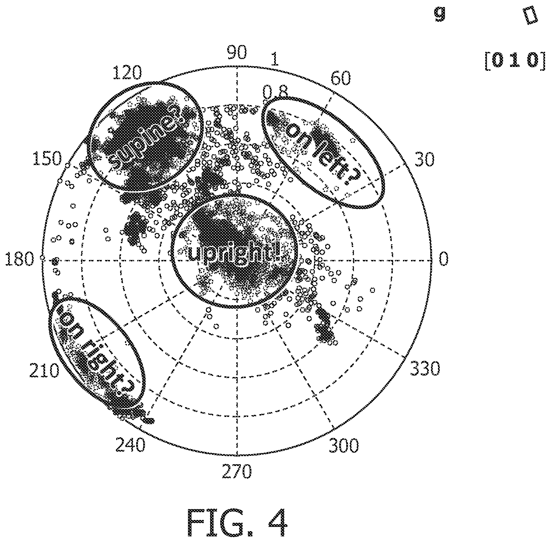

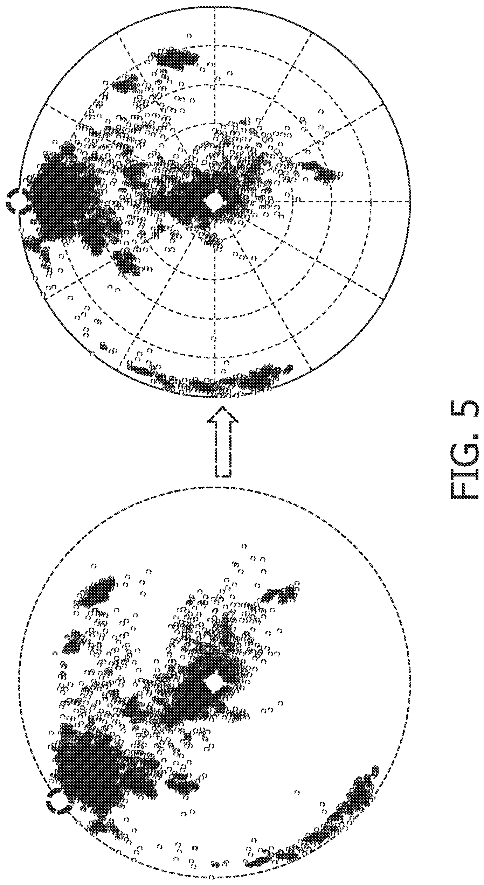

After all data points have been rotated such that the identified upright posture is at (0, 1, 0), the three-dimensional Cartesian view may be transformed into a two-dimensional polar view, as shown in Figure 4. The upright posture and thus y_{b} is located at the centre, so that y_{b} is pointing out of the plane of projection. Now, it remains to determine the position of the postures "on right", "on left", "supine" and "prone". Since it is very unlikely that a user spends a lot of time in an intermediate posture between "on a side" or "prone" and "upright", frequent intermediate postures near the upright posture should identify the direction of the supine posture. In other words, a posture change ending up in the upright posture will almost always start from the supine posture. In addition, a person will spend generally much more time in the supine posture than in the prone posture. These transitions are shown in the polar view of Figure 4 as data points located between the centre (upright posture) and the circumference of the unit g-sphere, since the supine posture should have an acceleration value of 1g (at rest), whereas posture transitions mostly have a value smaller than 1g. By identifying the most common angle or median angle outside a centre zone in the polar view and by rotating all points to align this angle with z_{b} corresponding to the supine posture in the b-system (x_{b}, y_{b}, z_{b}), the transformation of the orientation data measured in the a-system (xₐ, yₐ, zₐ) of the device into the b-system (x_{b}, y_{b}, z_{b}) of the body is completed. This is shown in Figure 5.

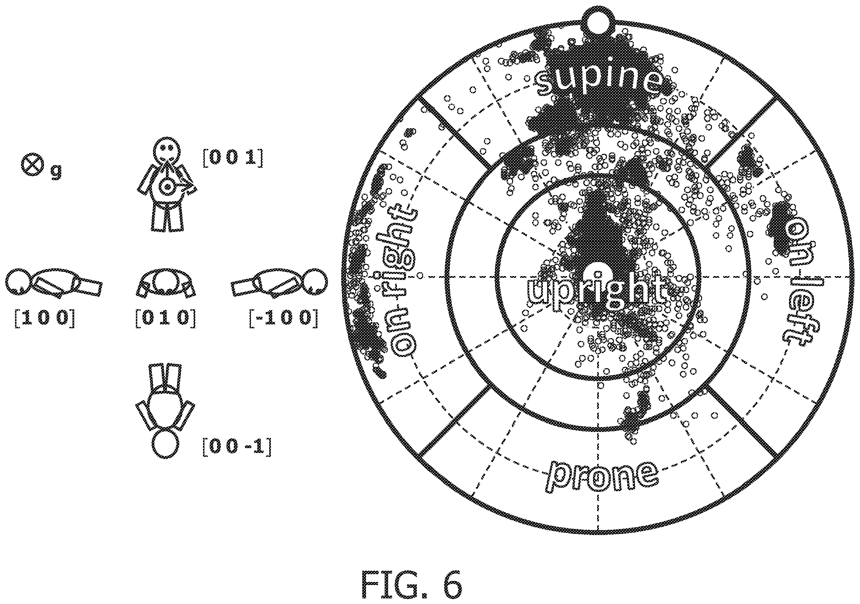

Figure 6 illustrates the aligned orientation data in a polar view of the b-system (x_{b}, y_{b}, z_{b}) of the body, wherein segments are drawn corresponding to the postures "upright", "prone", "on right", "supine" and "on left". A manikin is shown to illustrate the different postures with the respective three-dimensional coordinates of the b-system (x_{b}, y_{b}, z_{b}) in brackets. As indicated, gravity g is directed into the plane of projection.

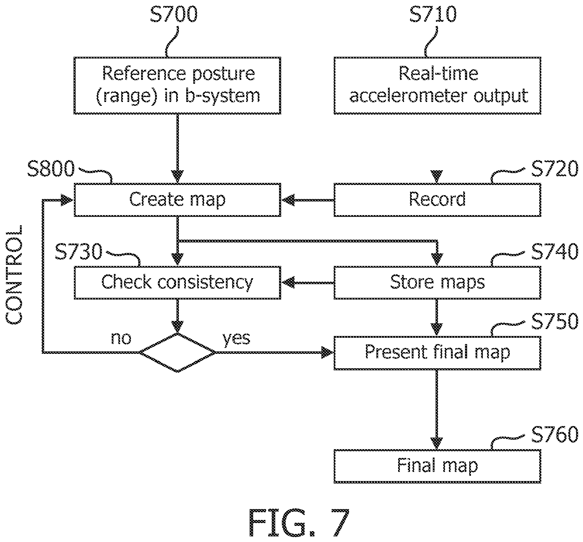

In Figure 7, another exemplary embodiment of the auto-calibration method according to the present invention is shown, wherein the reference condition comprises a reference posture or reference posture range defined in the b-system (x_{b}, y_{b}, z_{b}) and real-time accelerometer output is processed accordingly in order to determine the relative device orientation output as a "final map".

For this embodiment, the information on the general measurement situation has to be such, that the reference posture or posture range can be identified in advance and is input into the analysing system (S700). This reference posture (range) is required to have non-zero component(s) in x_{b} and/or z_{b}. Apart from being defined by value(s) in x_{b} and/or z_{b}, the reference posture (range) may be confined to a particular range in y_{b}. In the embodiment described in the following, this posture (range) has a uniquely high prevalence compared to other postures with the same component(s) in y_{b}. An example of such a reference posture range is the range of postures between "upright" and "supine", i.e. between y_{b} = 1 and z_{b} = 1. This example applies to the situation of hospital patients on general wards: here, this posture range can be expected to have a significantly higher prevalence than other postures with the same y_{b}-values, e.g. intermediate postures between "upright" and lying prone or on left or on right. Alternatively, the reference posture (range) may have a uniquely low prevalence or a unique prevalence pattern. However, in that case, the process "identify x_{b} in a-system" (S860) has to be adjusted.

Once the reference posture (range) has been identified as reference condition, it has to be registered (S700) into a calibration control software, which can run either on components inside the accelerometric device 1 or on an external device, such as a computer, or on both. In the latter two cases, some means for wired or wireless communication between the accelerometric device 1 and the computer system is required.

The layout of a calibration control routine is presented in Figure 7. Apart from the reference posture information, which may be entered in advance (S700), the only input to this system is the orientation data (S710). In this example, the orientation data relate to the real-time accelerometer output signals, which are produced, while the accelerometric device 1 is attached to the body 2. The signals are recorded for some time (S720), before feeding the recorded signals and the reference posture information to the "create map" process (S800). The map created by this process consists of the location of y_{b} and x_{b} in coordinates of the a-system. Instead of the location of x_{b}, the location of z_{b} may be contained in the calibration map. The choice for either is arbitrary, as the location of any third axis is fully defined by the location of the other two axes. Thus, a translation relation between the a-system of the device 1 and the b-system of the body 2 is determined in the "create map" process (S800). After creation, the map may be stored (S740). Optionally, the consistency may be checked (S730) against previously stored maps (if any), which were created earlier from shorter recordings. If sufficient consistency is found, the map is presented as the final map (S750) and the calibration is complete (S760). Otherwise, the recording of orientation data is continued for some time, after which a new map is created (S800). Instead of a system that produces a calibration map as soon as sufficient data is available and determines a final map in an iterative process, a system may be defined that produces such a map after data acquisition has been finished. This alternative would be useless in real-time posture detection applications, but would suffice in applications, where posture detection is only applied off-line. In a simpler embodiment, the calibration process may even only consist of a "create map" process (S800).

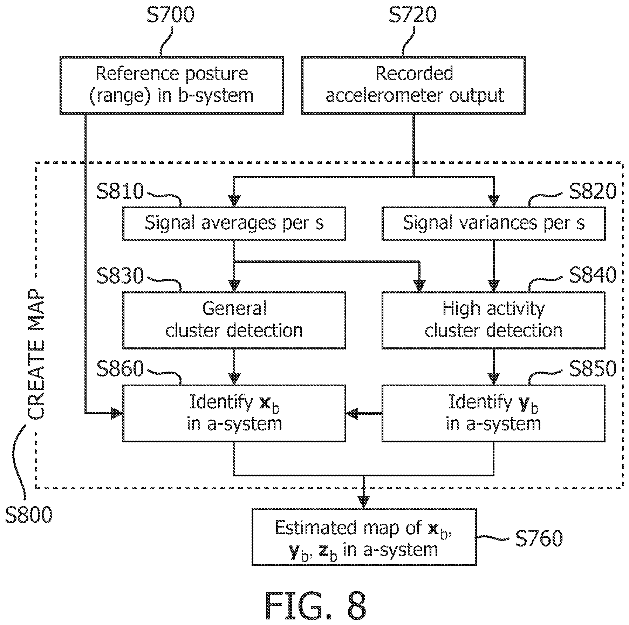

The layout of the "create map" process (S800) itself is presented in Figure 8. Its inputs are the reference posture information (S700) and the recorded accelerometer output (S720). The first steps are the calculation of averages (S810) and variances (S820) for orientation data in a certain time interval, e.g. for every second of data. The intervals may also be overlapping. Average can be taken as the average signal value per axis, and thus consists of (a vector of) three elements and variance can be taken as the average of the variances of the data of the three separate axes. Alternatively, the variance may be calculated as the variance of the magnitude of the vector sum of the signals from the three axes. This would imply that the contribution of rotations to the variance is suppressed. Obviously, any other measure for the signal amplitude may be used instead of the signal variance, e.g. the standard deviation. Furthermore, filtering steps may be performed before calculating the variances and averages (S820, S810) in order to selectively consider a specific frequency range. In another embodiment, the calculation of averages and variances (S810, S820) may be located at a higher level in the routine, i.e. before the recording step (S720) in the control routine. This alternative would require somewhat more real-time processing power, but would also save processing power in the "create map" process (S800) and would reduce the amount of data to be stored.

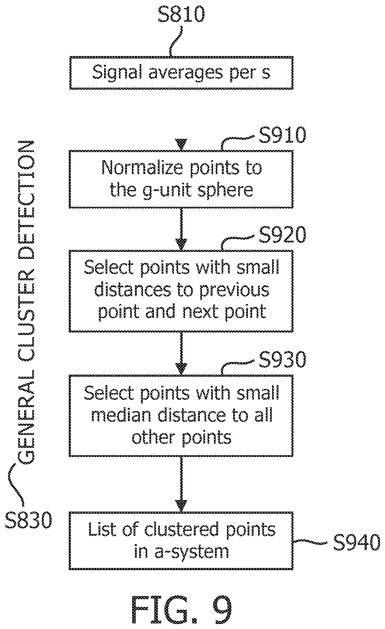

Within the "create map" process (S800), four other sub-processes are defined, the "general cluster detection" (S830), the "high activity cluster detection" (S840), the "identify x_{b} in a-system" (S860) and the "identify y_{b} in a-system" (S850), which are presented in Figures 9 to 13.

The layout of the "general cluster detection" sub-process (S830) is presented in Figure 9. First, the three-dimensional data average values (which constitute points in a three-dimensional space) are normalized (S910) to the surface of the unit g-sphere (i.e. the sphere with centre at the accelerometric zero point and radius of 1g). Optionally, a selection of points with a small distance to this surface may precede the normalization (S910), so that some points affected by large accelerations would be discarded. Then, points that have a small spatial distance to their previous and next point are selected (S920) in order to discard rapidly changing postures. This step (S920) may also be done before the normalization step (S910) or omitted. Finally, from the remaining points, the points are selected that have a small median distance to all other remaining points (S930). Distances may be generally calculated in various ways, e.g. as Euclidean distances or as distances in the unit g-sphere's surface. These selected points are considered to be clustered and are as such fed as a list of clustered points in the a-system of the device 1 (S940) to the "identify x_{b} in a-system" sub-process (S860).

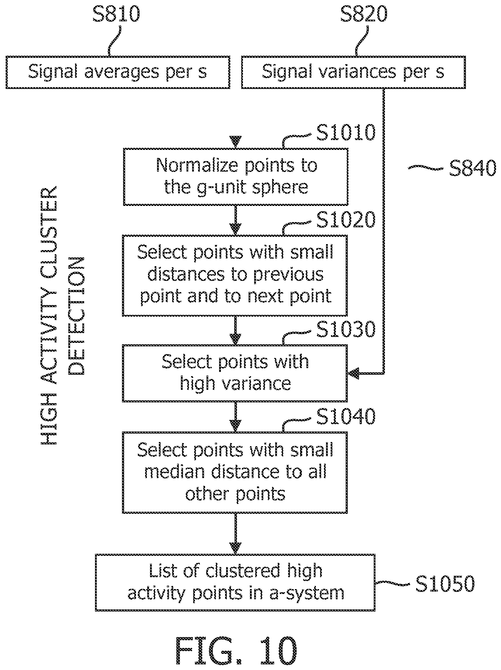

The layout of the "high activity cluster detection" sub-process (S840) is presented in Figure 10. In this sub-process (S840), the calculated signal averages (S810) and variances (S820) are input. The first two steps (S1010, S1020) in this sub-process (S840) are similar and possibly identical (depending on parameters) to those (S910, S920) in the "general cluster detection" sub-process (S830). Thus, if identical, these steps may also be combined for both sub-processes, i.e. steps S910 can be performed together with S1010 and likewise for S920 and S1020. In a further step (S1030), points with high variance are selected, based on the data variance information calculated in S820. Then, clusters are defined by selecting points with a small median distance to all other remaining points with high variance (S1040). This step (S1040) is again similar or identical (depending on parameters) to the final step (S940) in the "general cluster detection" sub-process (S840). The output of the "high activity cluster detection" sub-process (S840), which is a list of clustered high activity data points, is fed (S1050) to the "identify y_{b} in a-system" sub-process (S850).

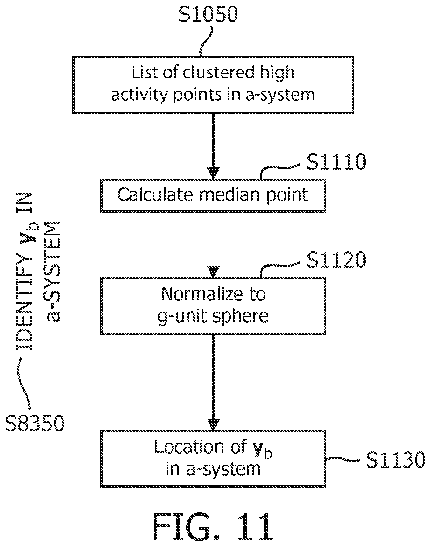

The layout of the "identify y_{b} in a-system" sub-process (S850) is presented in Figure 11. It starts with the calculation of a median point (or mean or average point) from the list of clustered high activity points (S1110). The median point is calculated as the medians of the three coordinates of all points, and is thus itself a point with a three-dimensional location. This median point is subsequently normalized to the unit g-sphere (S1120), before it is presented as the estimated location of y_{b} in the a-system (S1130) and output to the "identify x_{b} in a-system" sub-process (S860). Since the x_{b}-z_{b}-plane is defined as perpendicular to y_{b} and intersecting the accelerometric origin, the x_{b}-z_{b}-plane has also been identified in the "identify y_{b} in a-system" sub-process (S850).

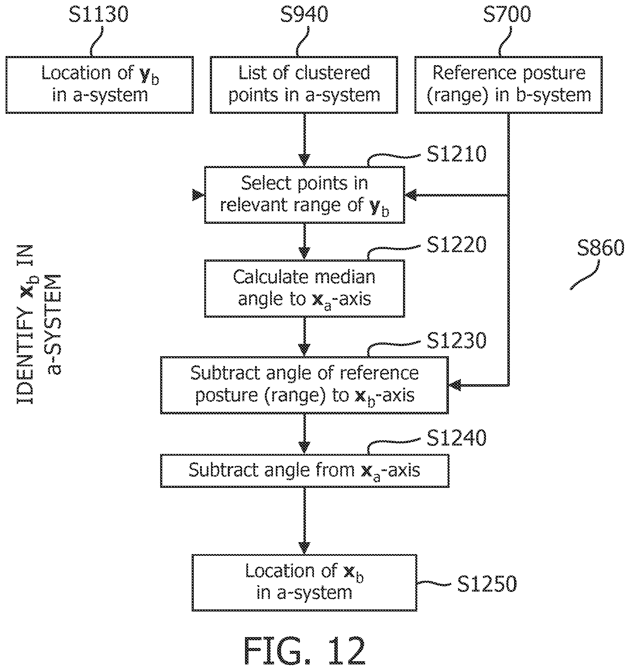

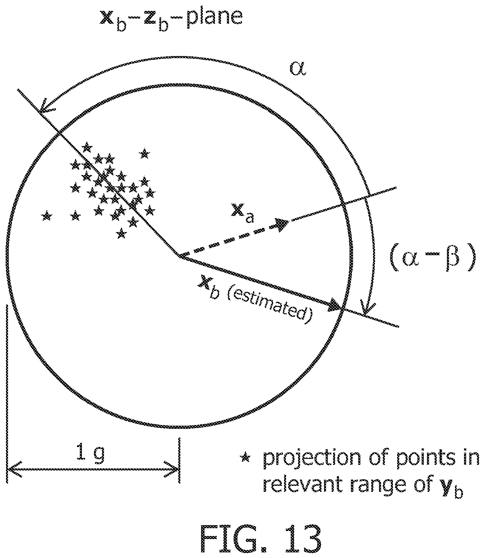

In order to align now the other two axes of the b-system with the respective axes of the a-system in the "identify x_{b} in a-system" sub-process (S860), the angle between x_{b} and the projection of xₐ on the x_{b}-z_{b}-plane has to be determined. The layout of the "identify x_{b} in a-system" sub-process (S860) is presented in Figure 12. First, using the estimated location of y_{b} in the a-system (S1130), those points from the list of clustered points in the a-system (output in S940) are selected (S1210) that belong to the y_{b} value (or to the range of y_{b} values), as specified by the reference posture (or reference posture range; S700). Instead of from the list of clustered points, the points may be selected alternatively from the list of all points or from a list of points selected based on intermediate selection criteria. Since these points in the relevant range of y_{b} should have non-zero components in x_{b} and/or z_{b}, as specified in the reference posture (range), they will not be located on the y_{b} axis itself, although they may be confined to a limited range of values in y_{b}. Thus, the median angle α between the projection of these points on the x_{b}-z_{b}-plane and the projection of xₐ on the x_{b}-z_{b}-plane is calculated (S1220). From this angle, the predefined angle β in the x_{b}-z_{b}-plane between the reference posture (range) and x_{b} is subtracted (S1230). The resulting angle is in turn subtracted in the x_{b}-z_{b}-plane from the projection of xₐ (S1240). The output (normalized to a magnitude of 1g) is the estimated location of x_{b} in the a-system (S1250). These steps are further illustrated in Figure 13.

Figure 13 shows the estimation of the location of x_{b} in the a-system, wherein α is the median angle between the projection of the points associated with the reference posture (range) on the x_{b}-z_{b}-plane and the projection of xₐ on the x_{b}-z_{b}-plane, and β is the predefined angle between the reference posture (range) and x_{b} in the b-system. However, instead of projecting xₐ on the x_{b}-z_{b}-plane, one can also project zₐ or a combination of xₐ and zₐ.

Having determined the translation relation between the a-system of the device 1 and the b-system of the body 2, the calibration of the device 1 is completed. By means of the translation relation, measured orientation data can now be transformed into the b-system of the body 2 for determining the body posture either in real-time or off-line.

The parameters of the method according to any one of the preceding embodiments, e. g. time intervals for calculating the average, median or variance etc., reference conditions, reference postures or reference posture ranges, constraints, assumptions, time intervals or conditions for repeating the calibration, etc., are preferably defined in advance and input by a user or supervisor. However, these parameters may also be defined during or after measuring the orientation data for off-line posture detection. Moreover, please note that the order of steps in the embodiments of the method according to the present invention is not intended to be compulsory. Thus, where appropriate and possible, the order of the steps may be changed. Generally, instead of medians, alternative measures of central value may be used, such as the average or mean. Likewise, instead of the variance, any alternative amplitude measure may be taken, e.g. the standard deviation. Various constraints (hard or soft) may be defined for the possible relative device orientation, which could enable more efficient and/or faster performing system designs. In the calibration control routine, steps may be incorporated to start processing only after specific criteria have been met in the recorded data (e.g. coverage time, range of postures, variance levels, etc.). Furthermore, other steps that are common to accompany data analysis in general may be included in one of the methods according to the present invention, such as filtering steps.

According to the present invention, an uncontrolled output of the accelerometer over a period of time together with a reference condition, such as signal amplitude measures (e. g. signal variance), may be sufficient to identify the relative orientation of the upright posture and to discriminate the relative orientation of the upright posture from the relative orientation of the horizontal postures (i.e. lying supine, on a side or prone). Furthermore, a signal amplitude measure in combination with a posture prevalence measure and/or other general information about the measurement situation may be sufficient to subsequently complete the calibration of the three-dimensional orientation of the body-mounted device 1 relative to the body 2. Thus, means are proposed for automatically calibrating a posture-detecting device without requiring skilled personnel, additional time or complex calibration requirements. The auto-calibration is particularly advantageous, when posture-detecting devices are used in general hospital wards, nursing homes or in private households, e.g. for posture detection by accelerometric devices used for monitoring the respiration rate or heart rate. However, the invention can be relevant in any situation where posture detection could be interesting. Examples include clinical alarming or information systems, patient monitoring in home situations, general fitness monitoring, ergonomics, physical therapy, rehabilitation training, sports and computer games. It should be noted that posture detection could play an important role in various context awareness applications. In theory, also applications could exist, in which posture detection is not of interest, but information about the relative device orientation is (e.g. because the relative location of vital organs is important). Also then, this invention could provide a solution.

## Claims

1. A device implantable or mountable to a body (2) for determining its orientation relative to the body (2), comprising:
- a three-dimensional orientation detection unit adapted to measure orientation data in a reference system of the orientation detection unit (xₐ, yₐ, zₐ); and
- a control unit adapted to determine the relative orientation of the device (1) with respect to the body (2) by determining a transformation relation between the body reference system (x_{b}, y_{b}, z_{b}) and the reference system of the orientation detection unit (xₐ, yₐ, zₐ);
**characterised in that** the control unit is adapted to automatically determine the transformation relation by means of at least one predetermined reference condition defined in a body reference system (x_{b}, y_{b}, z_{b}) and the measured orientation data, wherein the reference condition comprises a reference posture and/or a reference posture range defined in the body reference system (x_{b}, y_{b}, z_{b}) having at least one predetermined characteristic, the at least one predetermined characteristic relating to the prevalence level and/or activity level associated with the reference posture and/or with the reference posture range.

2. The device of claim 1, wherein the orientation detection unit comprises a three-dimensional accelerometer.

3. The device of claim 1 or 2, wherein the prevalence level and/or activity level of the reference posture relates to a high or low prevalence level and/or a high or low activity level with respect to other postures.

4. The device of claim 1, 2 or 3, wherein the control unit is further adapted to identify the reference posture and/or reference posture range in the measured orientation data by means of the predetermined characteristic in the reference system of the orientation detection unit (xₐ, yₐ, zₐ).

5. The device according to any one of the preceding claims, wherein at least one or more constraints and/or assumptions are definable for determining the orientation.

6. The device according to any one of the preceding claims, wherein the control unit is adapted to filter rapidly changing postures by setting a maximal spatial distance between subsequent data points.

7. The device according to any one of the preceding claims, wherein the control unit is further adapted to calculate at least one statistical parameter of the measured orientation data.

8. The device according to any one of the preceding claims, wherein the control unit is further adapted to perform cluster analysis on the measured orientation data and/or to process the measured orientation data separately for each axis of the reference system of the orientation detection unit (xₐ, yₐ, zₐ) resulting in three-dimensional values.

9. The device according to any one of the preceding claims, wherein the control unit is adapted to repeat the determination of the device orientation relative to the body (2) in predetermined time intervals and/or when triggered by a signal, and/or wherein the three-dimensional orientation detection unit is adapted to measure the orientation data continuously.

10. The device according to any one of the preceding claims, wherein the device is applicable for determining a posture of the body (2) by transforming the measured orientation data into the body reference system (x_{b}, y_{b}, z_{b}) using the determined transformation relation.

11. A system for determining an orientation of a device (1) relative to a body (2), comprising:
- a device (1) being implantable or mountable to the body (2) and having a three-dimensional orientation detection unit capable of measuring orientation data in a reference system of the orientation detection unit (xₐ, yₐ, zₐ); and
- a control unit capable of determining an orientation of the device (1) relative to the body (2) by determining a transformation relation between the body reference system (x_{b}, y_{b}, z_{b}) and the reference system of the orientation detection unit (xₐ, yₐ, zₐ);
**characterised in that** the control unit is adapted to automatically determine the transformation relation by means of a predetermined reference condition defined in a body reference system (x_{b}, y_{b}, z_{b}) and the measured orientation data wherein the reference condition comprises a reference posture and/or a reference posture range defined in the body reference system (x_{b}, y_{b}, z_{b}) having at least one predetermined characteristic, the at least one predetermined characteristic relating to prevalence level and/or activity level associated with the reference posture and/or with the reference posture range;
- wherein the control unit and the device (1) are in wired or wireless communication for unidirectional or bidirectional data transmission.

12. A method for determining an orientation of a body mounted or implanted device (1) relative to the body (2), the device (1) having a three-dimensional orientation detection unit, the method comprising:
- obtaining orientation data measured by the orientation detection unit in a reference system of the orientation detection unit (xₐ, yₐ, zₐ);
- defining at least one reference condition in a body reference system (x_{b}, y_{b}, z_{b}); and
- determining the orientation of the device (1) relative to the body (2) by determining a transformation relation between the body reference system (x_{b}, y_{b}, z_{b}) and the reference system of the orientation detection unit (xₐ, yₐ, zₐ);
**characterised in that** the control unit is adapted to automatically determine the transformation relation by means of the reference condition and the measured orientation data wherein the reference condition comprises a reference posture and/or a reference posture range defined in the body reference system (x_{b}, y_{b}, z_{b}) having at least one predetermined characteristic, the at least one predetermined characteristic relating to prevalence level and/or activity level associated with the reference posture and/or with the reference posture range.

13. A computer-readable medium useable with a computer and a device (1) that is implantable or mountable to a body (2) and comprises a three-dimensional orientation detection unit, the medium having a computer program stored, which when being executed by a processor, is adapted to carry out the method according to claim 12.

## Patentansprüche

1. An einem Körper (2) implantierbare oder montierbare Vorrichtung zum Ermitteln ihrer Orientierung in Bezug auf den Körper (2), die Folgendes umfasst:
- eine dreidimensionale Orientierungserkennungseinheit, die dafür ausgelegt ist, Orientierungsdaten in einem Referenzsystem der Orientierungserkennungseinheit (xₐ, yₐ, zₐ) zu messen; und
- eine Steuereinheit, die dafür ausgelegt ist, die relative Orientierung der Vorrichtung (1) in Bezug auf den Körper (2) zu ermitteln, indem sie eine Transformationsbeziehung zwischen dem Körperreferenzsystem (x_{b}, y_{b}, z_{b}) und dem Referenzsystem der Orientierungserkennungseinheit (xₐ, yₐ, zₐ) ermittelt;
**dadurch gekennzeichnet, dass** die Steuereinheit dafür ausgelegt ist, die Transformationsbeziehung mithilfe mindestens einer in dem Körperreferenzsystem (x_{b}, y_{b}, z_{b}) vorgegebenen Referenzbedingung und der gemessenen Orientierungsdaten automatisch zu ermitteln, wobei die Referenzbedingung eine in dem Körperreferenzsystem (x_{b}, y_{b}, z_{b}) definierte Referenzkörperhaltung und/oder einen Referenzkörperhaltungsbereich mit mindestens einer vorgegebenen Eigenschaft umfasst, wobei sich die mindestens eine vorgegebene Eigenschaft auf das zu der Referenzkörperhaltung und/oder zu dem Referenzkörperhaltungsbereich gehörige Prävalenzniveau und/oder Aktivitätsniveau bezieht.

2. Vorrichtung nach Anspruch 1, wobei die Orientierungserkennungseinheit einen dreidimensionalen Beschleunigungsmesser umfasst.

3. Vorrichtung nach Anspruch 1 oder 2, wobei sich das Prävalenzniveau und/oder Aktivitätsniveau der Referenzkörperhaltung auf ein hohes oder niedriges Prävalenzniveau und/oder ein hohes oder niedriges Aktivitätsniveau in Bezug auf andere Körperhaltungen bezieht.

4. Vorrichtung nach Anspruch 1, 2 oder 3, wobei die Steuereinheit weiterhin dafür ausgelegt ist, die Referenzkörperhaltung und/oder den Referenzkörperhaltungsbereich in den gemessenen Orientierungsdaten mithilfe der vorgegebenen Eigenschaft in dem Referenzsystem der Orientierungserkennungseinheit (xₐ, yₐ, zₐ) zu identifizieren.

5. Vorrichtung nach einem der vorhergehenden Ansprüche, wobei mindestens eine oder mehrere Einschränkungen und/oder Annahmen zum Ermitteln der Orientierung definierbar sind.

6. Vorrichtung nach einem der vorhergehenden Ansprüche, wobei die Steuereinheit dafür ausgelegt ist, sich schnell ändernde Körperhaltungen zu filtern, indem sie einen maximalen räumlichen Abstand zwischen aufeinanderfolgenden Datenpunkten einstellt.

7. Vorrichtung nach einem der vorhergehenden Ansprüche, wobei die Steuereinheit weiterhin dafür ausgelegt ist, mindestens einen statistischen Parameter der gemessenen Orientierungsdaten zu berechnen.

8. Vorrichtung nach einem der vorhergehenden Ansprüche, wobei die Steuereinheit weiterhin dafür ausgelegt ist, Cluster-Analysen für die gemessenen Orientierungsdaten durchzuführen und/oder die gemessenen Orientierungsdaten separat für jede Achse des Referenzsystems der Orientierungserkennungseinheit (xₐ, yₐ, zₐ) zu verarbeiten, wodurch sich dreidimensionale Werte ergeben.

9. Vorrichtung nach einem der vorhergehenden Ansprüche, wobei die Steuereinheit dafür ausgelegt ist, die Ermittlung der Vorrichtungsorientierung in Bezug auf den Körper (2) in vorgegebenen Zeitintervallen und/oder ausgelöst durch ein Signal zu wiederholen, und/oder wobei die dreidimensionale Orientierungserkennungseinheit dafür ausgelegt ist, die Orientierungsdaten kontinuierlich zu messen.

10. Vorrichtung nach einem der vorhergehenden Ansprüche, wobei die Vorrichtung zum Ermitteln einer Haltung des Körpers (2) verwendet werden kann, indem die gemessenen Orientierungsdaten unter Verwendung der ermittelten Transformationsbeziehung in das Körperreferenzsystem (x_{b}, y_{b}, z_{b}) transformiert werden.

11. System zur Ermittlung einer Orientierung einer Vorrichtung (1) in Bezug auf einen Körper (2), wobei das System Folgendes umfasst:
- eine Vorrichtung (1), die an einem Körper (2) implantierbar oder montierbar ist und eine dreidimensionale Orientierungserkennungseinheit hat, die in der Lage ist, Orientierungsdaten in einem Referenzsystem der Orientierungserkennungseinheit (xₐ, yₐ, zₐ) zu messen; und
- eine Steuereinheit, die in der Lage ist, eine Orientierung der Vorrichtung (1) in Bezug auf den Körper (2) zu ermitteln, indem sie eine Transformationsbeziehung zwischen dem Körperreferenzsystem (x_{b}, y_{b}, z_{b}) und dem Referenzsystem der Orientierungserkennungseinheit (xₐ, yₐ, zₐ) ermittelt;
**dadurch gekennzeichnet, dass** die Steuereinheit dafür ausgelegt ist, die Transformationsbeziehung mithilfe einer in einem Körperreferenzsystem (x_{b}, y_{b}, z_{b}) vorgegebenen Referenzbedingung und der gemessenen Orientierungsdaten automatisch zu ermitteln, wobei die Referenzbedingung eine in dem Körperreferenzsystem (x_{b}, y_{b}, z_{b}) definierte Referenzkörperhaltung und/oder einen Referenzkörperhaltungsbereich mit mindestens einer vorgegebenen Eigenschaft umfasst, wobei sich die mindestens eine vorgegebene Eigenschaft auf das zu der Referenzkörperhaltung und/oder zu dem Referenzkörperhaltungsbereich gehörige Prävalenzniveau und/oder Aktivitätsniveau bezieht;
wobei die Steuereinheit und die Vorrichtung (1) in einer drahtgebundenen oder drahtlosen Kommunikationsverbindung für die unidirektionale oder bidirektionale Datenübertragung stehen.

12. Verfahren zur Ermittlung einer Orientierung einer körpermontierten oder implantierten Vorrichtung (1) in Bezug auf einen Körper (2), wobei die Vorrichtung (1) eine dreidimensionale Orientierungserkennungseinheit hat, wobei das Verfahren Folgendes umfasst:
- Erlangen von durch die Orientierungserkennungseinheit in einem Referenzsystem der Orientierungserkennungseinheit (xₐ, yₐ, zₐ) gemessenen Orientierungsdaten;
- Definieren von mindestens einer Referenzbedingung in einem Körperreferenzsystem (x_{b}, y_{b}, z_{b}); und
- Ermitteln der Orientierung der Vorrichtung (1) in Bezug auf den Körper (2) durch Ermitteln einer Transformationsbeziehung zwischen dem Körperreferenzsystem (x_{b}, y_{b}, z_{b}) und dem Referenzsystem der Orientierungserkennungseinheit (xₐ, yₐ, zₐ);
**dadurch gekennzeichnet, dass** die Steuereinheit dafür ausgelegt ist, die Transformationsbeziehung mithilfe der Referenzbedingung und der gemessenen Orientierungsdaten automatisch zu ermitteln, wobei die Referenzbedingung eine in dem Körperreferenzsystem (x_{b}, y_{b}, z_{b}) definierte Referenzkörperhaltung und/oder einen Referenzkörperhaltungsbereich mit mindestens einer vorgegebenen Eigenschaft umfasst, wobei sich die mindestens eine vorgegebene Eigenschaft auf das zu der Referenzkörperhaltung und/oder zu dem Referenzkörperhaltungsbereich gehörige Prävalenzniveau und/oder Aktivitätsniveau bezieht.

13. Computerlesbares Medium, das mit einem Computer und einer Vorrichtung (1), die an einem Körper (2) implantierbar oder montierbar ist und eine dreidimensionale Orientierungserkennungseinheit umfasst, benutzbar ist, wobei das Medium ein gespeichertes Computerprogramm umfasst, das, wenn es durch einen Prozessor ausgeführt wird, dafür ausgelegt ist, das Verfahren nach Anspruch 12 durchzuführen.

## Revendications

1. Dispositif implantable ou montable sur un corps (2) pour déterminer son orientation par rapport au corps (2), comprenant :
- une unité de détection d'orientation tridimensionnelle adaptée pour mesurer des données d'orientation dans un système de référence de l'unité de détection d'orientation (xₐ, yₐ, zₐ) ; et
- une unité de commande adaptée pour déterminer l'orientation relative du dispositif (1) par rapport au corps (2) en déterminant une relation de transformation entre le système de référence du corps (x_{b}, y_{b}, z_{b}) et le système de référence de l'unité de détection d'orientation (xₐ, yₐ, zₐ) ;
**caractérisé en ce que** l'unité de commande est adaptée pour déterminer automatiquement la relation de transformation au moyen d'au moins une condition de référence prédéterminée définie dans un système de référence du corps (x_{b}, y_{b}, z_{b}) et des données d'orientation mesurées, dans lequel la condition de référence comprend une posture de référence et/ou une plage de posture de référence définie dans le système de référence du corps (x_{b}, y_{b}, z_{b}) possédant au moins une caractéristique prédéterminée, l'au moins une caractéristique prédéterminée concernant le niveau de prévalence et/ou le niveau d'activité associé à la posture de référence et/ou à la plage de posture de référence.

2. Dispositif selon la revendication 1, dans lequel l'unité de détection d'orientation comprend un accéléromètre tridimensionnel.

3. Dispositif selon la revendication 1 ou 2, dans lequel le niveau de prévalence et/ou niveau d'activité de la posture de référence se rapporte à un niveau de prévalence haut ou bas et/ou un niveau d'activité haut ou bas par rapport à d'autres postures.

4. Dispositif selon la revendication 1, 2 ou 3, dans lequel l'unité de commande est en outre adaptée pour identifier la posture de référence et/ou plage de posture de référence dans les données d'orientation mesurées au moyen de la caractéristique prédéterminée dans le système de référence de l'unité de détection d'orientation (xₐ, yₐ, zₐ).

5. Dispositif selon l'une quelconque des revendications précédentes, dans lequel au moins une ou plusieurs contraintes et/ou suppositions sont définissables pour déterminer l'orientation.

6. Dispositif selon l'une quelconque des revendications précédentes, dans lequel l'unité de commande est adaptée pour filtrer rapidement des postures changeantes en réglant une distance spatiale maximale entre des points de données suivants.

7. Dispositif selon l'une quelconque des revendications précédentes, dans lequel l'unité de commande est en outre adaptée pour calculer au moins un paramètre statistique des données d'orientation mesurées.

8. Dispositif selon l'une quelconque des revendications précédentes, dans lequel l'unité de commande est en outre adaptée pour réaliser une analyse de groupement sur les données d'orientation mesurées et/ou pour traiter les données d'orientation mesurées séparément pour chaque axe du système de référence de l'unité de détection d'orientation (xₐ, yₐ, zₐ) ayant pour résultat des valeurs tridimensionnelles.

9. Dispositif selon l'une quelconque des revendications précédentes, dans lequel l'unité de commande est adaptée pour répéter la détermination du dispositif orientation par rapport au corps (2) dans des intervalles de temps prédéterminés et/ou lors du déclenchement par un signal, et/ou dans lequel l'unité de détection d'orientation tridimensionnelle est adaptée pour mesurer les données d'orientation en continu.

10. Dispositif selon l'une quelconque des revendications précédentes, dans lequel le dispositif est applicable pour déterminer une posture du corps (2) en transformant les données d'orientation mesurées dans le système de référence du corps (x_{b}, y_{b}, z_{b}) en utilisant la relation de transformation déterminée.

11. Système pour déterminer une orientation d'un dispositif (1) par rapport à un corps (2), comprenant :
- un dispositif (1) implantable ou montable sur le corps (2) et possédant une unité de détection d'orientation tridimensionnelle capable de mesurer des données d'orientation dans un système de référence de l'unité de détection d'orientation (xₐ, yₐ, zₐ) ; et
- une unité de commande capable de déterminer une orientation du dispositif (1) par rapport au corps (2) en déterminant une relation de transformation entre le système de référence du corps (x_{b}, y_{b}, z_{b}) et le système de référence de l'unité de détection d'orientation (xₐ, yₐ, zₐ) ;
**caractérisé en ce que** l'unité de commande est adaptée pour déterminer automatiquement la relation de transformation au moyen d'une condition de référence prédéterminée définie dans un système de référence du corps (x_{b}, y_{b}, z_{b}) et des données d'orientation mesurées, dans lequel la condition de référence comprend une posture de référence et/ou une plage de posture de référence définie dans le système de référence du corps (x_{b}, y_{b}, z_{b}) possédant au moins une caractéristique prédéterminée, l'au moins une caractéristique prédéterminée se rapportant à un niveau de prévalence et/ou niveau d'activité associé à la posture de référence et/ou à la plage de posture de référence ;
dans lequel l'unité de commande et le dispositif (1) sont en communication câblée ou sans fil pour la transmission de données unidirectionnelle ou bidirectionnelle.

12. Procédé pour déterminer une orientation d'un dispositif monté ou implanté sur corps (1) par rapport au corps (2), le dispositif (1) possédant une unité de détection d'orientation tridimensionnelle, le procédé comprenant :
- l'obtention de données d'orientation mesurées par l'unité de détection d'orientation dans un système de référence de l'unité de détection d'orientation (xₐ, yₐ, zₐ) ;
- la définition d'au moins une condition de référence dans un système de référence du corps (x_{b}, y_{b}, z_{b}) ; et
- la détermination de l'orientation du dispositif (1) par rapport au corps (2) en déterminant une relation de transformation entre le système de référence du corps (x_{b}, y_{b}, z_{b}) et le système de référence de l'unité de détection d'orientation (xₐ, yₐ, zₐ) ;
**caractérisé en ce que** l'unité de commande est adaptée pour déterminer automatiquement la relation de transformation au moyen de la condition de référence et des données d'orientation mesurées, dans lequel la condition de référence comprend une posture de référence et/ou une plage de posture de référence définie dans le système de référence du corps (x_{b}, y_{b}, z_{b}) possédant au moins une caractéristique prédéterminée, l'au moins une caractéristique prédéterminée se rapportant à un niveau de prévalence et/ou niveau d'activité associé à la posture de référence et/ou à la plage de posture de référence.

13. Support lisible par ordinateur utilisable avec un ordinateur et un dispositif (1) qui est implantable ou montable sur un corps (2) et comprend une unité de détection d'orientation tridimensionnelle, le support possédant un programme d'ordinateur stocké, qui, lorsqu'il est exécuté par un processeur, est adapté pour effectuer le procédé selon la revendication 12.
